# EUROPÄISCHE PATENTSCHRIFT

(11) **EP 2 485 828 B1**
(45) Veröffentlichungstag und Bekanntmachung des Hinweises auf die Patenterteilung: **13.06.2018**
(21) Anmeldenummer: 10768402.9
(22) Anmeldetag: 01.10.2010
(51) Int. Cl.: B01D 61/22, B01D 65/10

(54) **VORRICHTUNG UND VERFAHREN ZUR ÜBERPRÜFUNG EINES FILTERS FÜR EINE EXTRAKORPORALE BLUTBEHANDLUNGSVORRICHTUNG**
DEVICE AND METHOD FOR INSPECTING A FILTER FOR AN EXTRACORPOREAL BLOOD TREATMENT DEVICE
DISPOSITIF ET PROCÉDÉ DE CONTRÔLE D'UN FILTRE POUR UN DISPOSITIF DE TRAITEMENT SANGUIN EXTRACORPOREL

(30) Priorität: 10.10.2009 DE 102009048920
(43) Veröffentlichungstag der Anmeldung: 15.08.2012
(73) Patentinhaber: Fresenius Medical Care Deutschland GmbH, 61352 Bad Homburg v.d.H. (DE)
(72) Erfinder: KOPPERSCHMIDT, Pascal, 97456 Dittelbrunn (DE); BOCKLET, Christoph, 97708 Bad Bocklet (DE)
(74) Vertreter: Oppermann, Frank
(86) Internationale Anmeldenummer: PCT/EP2010/006013
(87) Internationale Veröffentlichungsnummer: WO 2011/042137

(56) Entgegenhaltungen:
- EP-A1- 0 911 043
- WO-A1-2004/089440
- WO-A1-2006/026011
- US-A- 5 057 212
- US-A- 5 644 240
- US-A- 5 779 911

## Beschreibung

Die Erfindung betrifft eine Vorrichtung und ein Verfahren zur Überprüfung eines Filters für eine extrakorporale Blutbehandlungsvorrichtung, insbesondere eines Filters zum Filtern von Dialysierflüssigkeit für eine extrakorporale Blutbehandlungsvorrichtung.

Es sind verschiedene Verfahren zur extrakorporalen Blutbehandlung bekannt. Bei der Hämodialyse (HD) wird das Blut des Patienten in einem extrakorporalen Blutkreislauf gereinigt, der einen Dialysator umfasst. Der Dialysator weist eine Blutkammer und eine Dialysierflüssigkeitskammer auf, die von einer semipermeablen Membran getrennt sind.

Während bei der Hämodialyse (HD) die Dialysierflüssigkeitskammer von Dialysierflüssigkeit durchflossen wird, wobei bestimmte Substanzen aufgrund der Diffusion zwischen der Dialysierflüssigkeit und dem Blut durch die Membran transportiert werden, wird bei der Hämofiltration (HF) die Dialysierflüssigkeitskammer des Dialysators nicht von Dialysierflüssigkeit durchströmt. Bei der Hämofiltration (HF) werden bestimmte Substanzen aufgrund von Konvektion durch die Membran des Filters effektiv entfernt. Eine Kombination aus beiden Verfahren ist die Hämodiafiltration (HDF).

Es ist allgemein bekannt, einen Teil der dem Patienten über die Membran des Dialysators oder Filters entzogenen Flüssigkeit durch eine sterile Substitutionsflüssigkeit (Substituat) zu ersetzen, die entweder stromauf oder stromab des Dialysators dem extrakorporalen Blutkreislauf zugeführt wird. Es sind Vorrichtungen zur extrakorporalen Blutbehandlung bekannt, bei denen die Dialysierflüssigkeit online aus Frischwasser und Konzentrat und das Substituat online aus der Dialysierflüssigkeit hergestellt werden. Das Substituat wird dem extrakorporalen Blutkreislauf aus dem Dialysierflüssigkeitssystem der Blutbehandlungsvorrichtung über eine Substituatleitung stromauf oder stromab der Blutkammer des Dialysators zugeführt.

Um eine eventuelle Kontamination der Dialysierflüssigkeit mit Giftstoffen (Pyrogenen) zu vermeiden, wird die Dialysierflüssigkeit gefiltert, bevor sie dem Blutkreislauf zugeführt wird. Hierzu verfügen die bekannten Blutbehandlungsvorrichtungen über spezielle Pyrogenfilter, die zur Wahrung eines pyrogenfreien Dialysats (Permeats) Abbauprodukte von Bakterien und Endotoxine wirkungsvoll zurückhalten.

Die bekannten Blutbehandlungsvorrichtungen verfügen im Allgemeinen über ein oder mehrere in Serie geschaltete Pyrogenfilter, die im Flüssigkeitssystem angeordnet sind. Die Pyrogenfilter haben einen ähnlichen Aufbau wie der Dialysator. Es sind Pyrogenfilter bekannt, die über einen Bündel von Hohlfasern verfügen. Derartige Pyrogenfilter werden auch als Kapillarfilter bezeichnet. Das Hohlfaserbündel ist in einem zylindrischen Gehäuse derart angeordnet, dass die Öffnungen der einzelnen Fasern an den Stirnseiten des Bündels freiliegen. Die Kapillaren in den Hohlfasern bilden die Primärseite und der Zwischenraum zwischen den Hohlfasern die Sekundärseite des Filters. Die Filterung erfolgt diffusiv durch Druckunterschied transversal zur Faserrichtung.

Es ist möglich, die Pyrogenfilter einem initialen Drucktest mit komprimierter Luft zu unterziehen. Hierbei wird der Filter stromab durch ein Ventil geschlossen und stromauf mit einem Luftüberdruck beaufschlagt und der Druck wird überwacht. Bleibt der Druck über eine gewisse Zeitspanne unverändert, kann von einem intakten Filter ausgegangen werden. Sinkt der Druck, so lässt das auf Undichtigkeiten der Fasern schließen, durch die die komprimierte Luft entweichen kann.

Die bekannten Pyrogenfilter müssen nach einer gewissen Standzeit ausgewechselt werden. Nachteilig ist, dass in der Praxis die Wechselintervalle für die Pyrogenfilter unabhängig von dem tatsächlichen Zustand des Filters fest vorgegeben werden müssen. In der Praxis können daher die Filter grundsätzlich zu früh oder zu spät gewechselt werden.

Die US 2008/0203023 A1 beschreibt eine Blutbehandlungsvorrichtung, die über einen Pyrogenfilter verfügt, der einen integrierten Leitfähigkeitssensor aufweist, um die Qualität der durch den Filter strömenden Dialysierflüssigkeit zu überwachen. Damit allein kann aber noch keine Aussage über den Zustand des Filters getroffen werden.

Die WO 2008/089913 A2 beschreibt eine Blutbehandlungsvorrichtung, die über eine Einrichtung zur Feststellung der Verkalkung eines Pyrogenfilters verfügt. Diese Einrichtung weist Sensoren auf, die stromauf und stromab des Filters angeordnet sind. Die Sensoren messen die Leitfähigkeit der durch die semipermeable Membran des Filters kontinuierlich strömenden Dialysierflüssigkeit.

Die DE 198 32 451 C1 beschreibt eine Vorrichtung zum Überprüfen des ordnungsgemäßen Austauschs eines gebrauchten Filters in einer Vorrichtung zur extrakorporalen Blutbehandlung. Die Überprüfung des ordnungsgemäßen Filterwechsels erfolgt mittels eines Druckhaltetests, wobei für den Fall der Durchlässigkeit der Membran des Filters für Gas darauf geschlossen wird, dass der gebrauchte Filter gegen einen neuen Filter ausgetauscht worden ist.

Die WO 2004/089440 A1 beschreibt eine Blutbehandlungsvorrichtung mit einem Dialysierflüssigkeitssystem, das einen Sterilfilter umfasst, der durch eine semipermeable Membran in eine erste und zweite Kammer unterteilt ist. In den Flüssigkeitsleitungen stromab der beiden Kammern des Sterilfilters befinden sich Leitfähigkeitssensoren. Darüber hinaus sind Ventile in den Flüssigkeitsleitungen des Dialysierflüssigkeitssystems vorgesehen, mit denen eine longitudinale Flüssigkeitsströmung entlang der Membran oder eine transversale Flüssigkeitsströmung durch die Membran des Sterilfilters hergestellt werden kann. Mit einer longitudinalen Durchströmung des Sterilfilters soll die Filtermembran gespült werden. Die Leitfähigkeitssensoren sind zur Messung der Natriumionenkonzentration bestimmt. Die Steuer- und Auswerteinheit der bekannten Blutbehandlungsvorrichtung ist derart konfiguriert, dass die Blutreinigungsleistung bestimmt wird. Hierzu steuert die Steuer- und Recheneinheit die Ventile an und nimmt Messwerte von den Leitfähigkeitssensoren im Dialysierflüssigkeitssystem auf.

Aus der US 5,779,911 A ist ein Wasseraufbereitungssystem mit einem Filter bekannt, der durch eine Membran in zwei Kammern unterteilt ist. Zur Überwachung der Wasseraufbereitung sind Leitfähigkeitssensoren im Flüssigkeitssystem vorgesehen. Die Steuerung der Flüssigkeitsströmung erfolgt mit Ventilen im Flüssigkeitssystem.

Die EP 0 911 043 A1 beschreibt ebenfalls eine Blutbehandlungsvorrichtung, die eine Leitfähigkeitsmessung im Dialysierflüssigkeitssystem vorsieht.

Der Erfindung liegt die Aufgabe zugrunde, eine Vorrichtung zur Überprüfung eines Filters für eine extrakorporale Blutbehandlungsvorrichtung, insbesondere eines Filters zum Filtern von Dialysierflüssigkeit, zu schaffen, mit der sich der ordnungsgemäße Zustand des Filters sicher feststellen lässt. Eine weitere Aufgabe der Erfindung ist, eine extrakorporale Blutbehandlungsvorrichtung mit einer derartigen Vorrichtung bereitzustellen und ein Verfahren zur Überprüfung eines Filters anzugeben.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung und das erfindungsgemäße Verfahren zur Überprüfung eines Filters beruhen auf der Messung des Strömungspotentials vor und nach dem Wechsel der Flüssigkeitsströmung einer elektrolytischen Flüssigkeit, insbesondere Permeat (Reinstwasser) oder Dialysierflüssigkeit, zwischen einem transversalen Fluss durch die semipermeable Membran des Filters und eines longitudinalen Flusses entlang der semipermeablen Membran des Filters. Es ist aber alternativ auch möglich, das Strömungspotential der elektrolytischen Flüssigkeit vor und nach dem Wechsel zwischen einem longitudinalen Fluss entlang der semipermeablen Membran des Filters und einem transversalen Flusses durch die semipermeable Membran des Filters zu messen. Grundsätzlich ist es auch möglich, die Leitfähigkeiten sowohl bei einem Übergang von einem transversalen auf einen longitudinalen Fluss als auch bei einem Übergang von einem longitudinalen Fluss auf einen transversalen Fluss zu messen.

Die Erfassung des Strömungspotentials durch eine Leitfähigkeitsmessung erlaubt die nicht-invasive Überprüfung der Integrität eines Filters, insbesondere Pyrogenfilters. Durch die Nutzung des Filters ändert sich dessen Endotoxin-Rückhalterate infolge einer veränderten Permeabilität der Filterporen. Dies führt zu einer Änderung der Größe des Strömungspotentials. Die Größe des Strömungspotentials hängt des Weiteren von der Elektrolytkonzentration, der Elektrolytart, der Strömungsgeschwindigkeit, der Oberflächenpolarität und der Geometrie der Poren des Filters ab.

Nach der Änderung der Strömungsrichtung der elektrolytischen Flüssigkeit zeigt sich eine Änderung der Leitfähigkeit nach dem Filter in transversaler oder longitudinaler Richtung ausgehend von einem Basiswert auf einen höheren Wert oder einen niedrigeren Wert, wobei sich nach einem bestimmten Zeitintervall die Leitfähigkeit wieder auf den Basiswert einstellt. Ein nicht ordnungsgemäßer Zustand des Filters wird auf der Grundlage der Änderung der Leitfähigkeit nach dem Wechsel der Strömungsrichtung der elektrolytischen Flüssigkeit festgestellt.

Die erfindungsgemäße Vorrichtung verfügt zur Durchführung des Messverfahrens über Mittel zum Erzeugen eines Wechsels der Strömungsrichtung von einem transversalen auf einen longitudinalen und/oder von einem longitudinalen auf einen transversalen Fluss. Derartige Mittel können dem Fachmann bekannte Flüssigkeitsleitungen und Absperrorgane umfassen, mit denen die elektrolytische Flüssigkeit entlang der Membran oder durch die Membran geleitet wird. Darüber hinaus verfügt die erfindungsgemäße Vorrichtung über eine Rechen- und Auswerteinheit zur Überwachung der Änderung der Leitfähigkeit nach dem Wechsel der Strömungsrichtung der elektrolytischen Flüssigkeit, die derart ausgebildet ist, dass ein nicht ordnungsgemäßer Zustand des Filters auf der Grundlage der Änderung der Leitfähigkeit nach dem Wechsel der Strömungsrichtung der elektrolytischen Flüssigkeit festgestellt wird.

Die Mittel zum Messen der Leitfähigkeit können einen Leitfähigkeitssensor umfassen, der stromab des Filters auf der Primärseite angeordnet ist, oder einen Leitfähigkeitssensor umfassen, der stromab des Filters auf der Sekundärseite des Filters angeordnet ist. Hierbei umfasst die Primärseite des Filters das Innere der Hohlfasern, an die der Flüssigkeitszulauf angeschlossen wird, die Sekundärseite den Bereich zwischen dem Dialysatorgehäuse und den Hohlfasern, in dem sich die durch die Poren der Hohlfasern gefilterte Flüssigkeit ansammelt. Es ist aber auch möglich, dass zwei Sensoren vorgesehen sind, von denen der eine auf der Primärseite und der andere auf der Sekundärseite angeordnet ist. Anstelle von zwei Sensoren zur Erfassung der Leitfähigkeit sowohl auf der Primär- als auch Sekundärseite kann auch nur ein einziger Leitfähigkeitssensor vorgesehen sein, wenn über eine dem Fachmann bekannte Anordnung von Flüssigkeitsleitungen und Absperrorganen die elektrolytische Flüssigkeit für beide Messungen dem einzigen Leitfähigkeitssensor zugeführt wird.

Eine bevorzugte Ausführungsform der Erfindung sieht vor, nach einem Wechsel der Strömungsrichtung von einem transversalen Fluss auf einen longitudinalen Fluss den Betrag des Anstiegs der Leitfähigkeit der elektrolytischen Flüssigkeit nach dem Filter in longitudinaler Richtung von einem Basiswert auf einen Maximalwert zu ermitteln, d.h. die Höhe des nachzuweisenden Leitfähigkeitssprunges zu detektieren. Die Höhe des Leitfähigkeitssprunges hängt von der Basisleitfähigkeit des Elektrolyts und von der Polarisation der Membran ab. Der Betrag des Leitfähigkeitsanstiegs wird mit einem vorgegebenen Grenzwert verglichen, wobei ein nicht ordnungsgemäßer Zustand des Filters festgestellt wird, wenn der Betrag kleiner als der vorgegebene Grenzwert ist.

Eine alternative Ausführungsform sieht vor, eine Änderung der Leitfähigkeit der elektrolytischen Flüssigkeit nach dem Filter in transversaler Richtung nach einem Wechsel von einem longitudinalen Fluss auf einen transversalen Fluss zu überwachen. In diesem Fall kann ein Abfall der Leitfähigkeit von dem Basiswert auf einen Minimalwert um einen bestimmten Betrag nachgewiesen werden. Die Höhe des Leitfähigkeitsabfalls hängt von der Basisleitfähigkeit des Elektrolyts und von der Polarisation der Membran ab. Der Betrag des Leitfähigkeitsabfalls wird mit einem vorgegebenen Grenzwert verglichen, wobei ein nicht ordnungsgemäßer Zustand des Filters festgestellt wird, wenn der Betrag kleiner als der vorgegebene Grenzwert ist. Grundsätzlich ist es auch möglich, beide alternativen Ausführungsformen miteinander zu kombinieren, wobei die ermittelten Messwerte mit für den Fachmann bekannten Verfahren statistisch ausgewertet werden.

Eine weitere alternative Ausführungsform sieht anstelle der Ermittlung des Betrages der Leitfähigkeitsveränderungen von einem Basiswert die Bestimmung der Integrale über die Zeit der Leitfähigkeitsveränderungen vor. Dabei zeigt sich, dass das Integral des Leitfähigkeitsprungs bei einem Wechsel von einem transversalen zu einem longitudinalen Fluss aus Gründen der Ladungserhaltung gleich dem Integral des Leitfähigkeitsabfalls bei einem Wechsel von einem longitudinalen Fluss auf einen transversalen Fluss ist.

Es hat sich gezeigt, dass die Größe der Leitfähigkeitsveränderung, bzw. deren Interal über die Zeit, abnimmt, wenn sich die Poren der semipermeablen Membran des Filters mit zunehmender Nutzungsdauer zusetzen. Damit kann mit einem bestimmten Grenzwert die maximal zulässige Standzeit des Filters vorgegeben werden. Wenn der Betrag der Leitfähigkeitsveränderung oder deren Integral über die Zeit kleiner als der vorgegebene Grenzwert ist, muss der Filter ausgetauscht werden. Der vorgegebene Grenzwert kann in einem Speicher der Rechen- und Auswerteeinheit abgelegt sein. Der Grenzwert kann empirisch mit Vergleichsmessungen ermittelt werden. Es ist möglich, den Grenzwert dann festzulegen, wenn ein neuer Filter eingesetzt wird, wobei der Grenzwert dann ein bestimmter prozentualer Anteil des Betrags der Erhöhung der Leitfähigkeit ist, der mit dem neuen Filter gemessen wird.

Mit dem erfindungsgemäßen Verfahren und der erfindungsgemäßen Vorrichtung kann nicht nur festgestellt werden, wann der Filter ausgetauscht werden muss, sondern es ist auch möglich, festzustellen, ob in eine extrakorporale Blutbehandlungsvorrichtung ein Ersatzfilter eingesetzt ist, der nur dem Kurzschluss der Flüssigkeitsleitungen dient, aber keine Filterwirkung hat. Auf den Einsatz eines Ersatzfilters wir dann geschlossen, wenn sich eine Leitfähigkeitsänderung nicht nachweisen lässt.

Darüber hinaus ist es möglich, die Integrität der Kapillaren der semipermeablen Membran des Filters mit der erfindungsgemäßen Vorrichtung und dem erfindungsgemäßen

Verfahren zu überprüfen. Auf Risse oder Defekte in der semipermeablen Membran kann dann geschlossen werden, wenn sich ein signifikanter Leitfähigkeitssprung nach dem Wechsel der Strömungsrichtung nicht nachweisen lässt.

Wenn in der extrakorporalen Blutbehandlungsvorrichtung zum Filtern der Dialysierflüssigkeit mehrere Filter vorhanden sind, können sämtliche Filter mit der erfindungsgemäßen Vorrichtung und dem erfindungsgemäßen Verfahren auf einen ordnungsgemäßen Zustand überprüft werden. Es ist auch möglich, die Änderungen der Leitfähigkeit, die sich bei beiden Filtern ergeben, miteinander zu vergleichen, um eine Aussage über den Zustand des einen Filters im Vergleich zu dem Zustand des anderen Filters treffen zu können.

Im Folgenden werden mehrere Ausführungsbeispiele der Erfindung unter Bezugnahme auf die Zeichnungen näher erläutert.

Es zeigen:
- Fig. 1: Die wesentlichen Komponenten einer Vorrichtung zur extrakorporalen Blutbehandlung mit einer Vorrichtung zur Überprüfung der Filter zum Filtern der Dialysierflüssigkeit in stark vereinfachter schematischer Darstellung,
- Fig. 2: die Änderung der Leitfähigkeit der Dialysierflüssigkeit stromab des Filters nach einem Wechsel der Strömungsrichtung zwischen einem transversalen und einem longitudinalen Fluss und einem longitudinalen und transversalen Fluss,
- Fig. 3: den Aufbau eines Kapillarfilters zum Filtern von Dialysierflüssigkeit und
- Fig. 4: die Veränderung der Leitfähigkeit mit der Anzahl der Entfettungszyklen eines Pyrogenfilters.

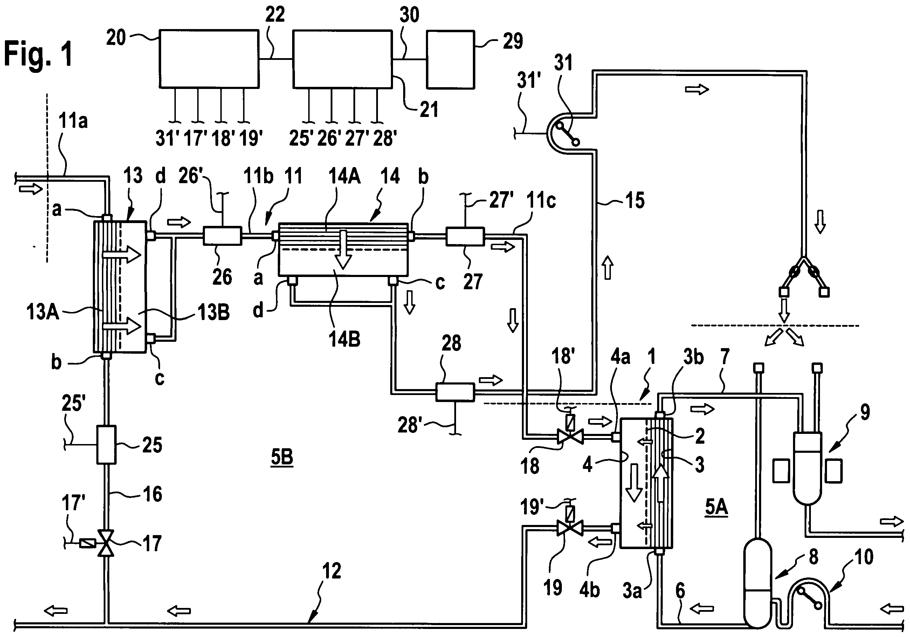

Fig. 1 zeigt eine vereinfachte schematische Darstellung der wesentlichen Komponenten einer extrakorporalen Blutbehandlungsvorrichtung, insbesondere einer Hämo(dia)filtrationsvorrichtung, die über eine Vorrichtung zur Überprüfung der in der Blutbehandlungsvorrichtung vorhandenen Filter zum Filtern der Dialysierflüssigkeit verfügt.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer in das Flüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt und eine venöse Blutleitung 7, die von dem Auslass 3b der Blutkammer des Dialysators 1 abgeht. Zur Eliminierung von Luftblasen sind bei dem vorliegenden Ausführungsbeispiel in die arterielle Blutleitung 6 eine arterielle Tropfkammer 8 und in die venöse Blutleitung 7 eine venöse Tropfkammer 9 geschaltet. Das Blut wird im extrakorporalen Kreislauf mit einer Blutpumpe 10 gefördert, die in der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer 4, während verbrauchte Dialysierflüssigkeit aus der Dialysierflüssigkeitskammer über die Dialysierflüssigkeitsabführleitung 12 zu einem nicht dargestellten Abfluss abgeführt wird. Die Dialysierflüssigkeit wird mit einer nicht dargestellten Dialysierflüssigkeitspumpe im Flüssigkeitssystem 5B gefördert.

Während der Blutbehandlung kann eine sterile Substitutionsflüssigkeit (Substituat) aus der Dialysierflüssigkeit im Flüssigkeitssystem 5B gewonnen und dem extrakorporalen Blutkreislauf 5A zugeführt werden. Zur Gewinnung steriler Dialysierflüssigkeit dienen zwei Sterilfilter 13, 14 (Pyrogenfilter), die im Flüssigkeitssystem 5B angeordnet sind. Bei beiden Sterilfiltern handelt es sich um Kapillarfilter, deren semipermeable Membran von einem Bündel von Hohlfasern gebildet wird.

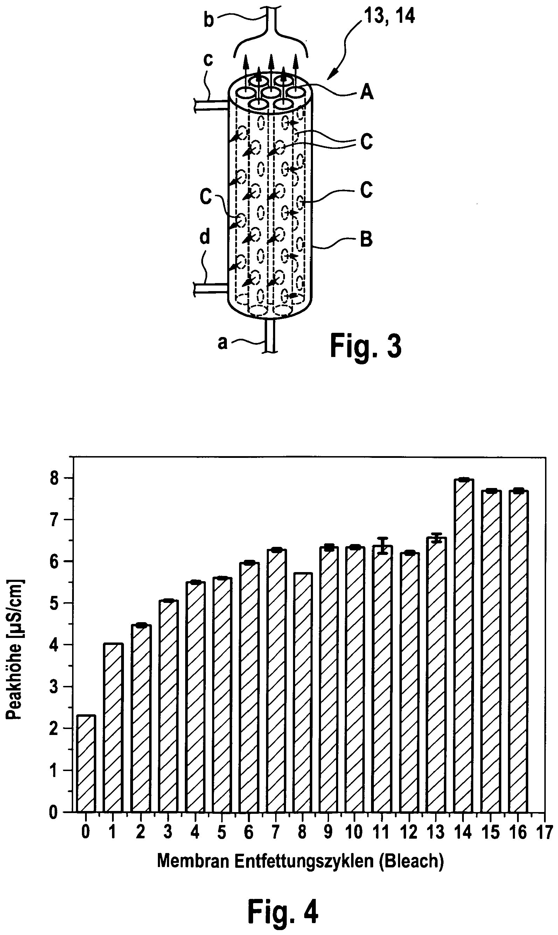

Fig. 3 zeigt in stark vereinfachter schematischer Darstellung den Aufbau eines der beiden Sterilfilter 13, 14 (Pyrogenfilter). Der Hohlfaserbündel A ist in einem zylindrischen Gehäuse B derart angeordnet, dass die Öffnungen der einzelnen Fasern an den Stirnseiten des Gehäuses freiliegen. An den Stirnseiten des Gehäuses befinden sich an den Öffnungen der Kapillaren die beiden Anschlüsse a und b der ersten Kammer (Primärseite) des Filters, während an dem oberen und unteren Ende der Gehäusewand sich die beiden Auslässe c und d der zweiten Kammer (Sekundärseite) des Filters befinden. Wenn zwischen der Primär- und Sekundärseite ein Druckunterschied besteht, findet ein Austausch durch die Poren C des Hohlfaserbündels A statt. In Fig. 3 ist die Strömungsrichtung durch die Poren der Kappilaren des Hohlfaserbündels A in transversaler Richtung und entlang der Kappilaren in longitudinaler Richtung durch Pfeile gekennzeichnet.

Die beiden Sterilfilter 13, 14 sind im Flüssigkeitssystem 5B in Serie geschaltet. Ein erster Abschnitt 11a der Dialysierflüssigkeitszuführleitung 11 führt zu dem ersten Einlass a der ersten Kammer 13A des ersten Sterilfilters 13. Von den beiden Auslässen c, d der zweiten Kammer 13B des ersten Sterilfilters 13 geht ein zweiter Abschnitt 11b der Dialysierflüssigkeitszuführleitung 11 ab und führt zu dem einen Einlass a der ersten Kammer 14A des zweiten Sterilfilters 14. Von dem zweiten Einlass b der ersten Kammer 14A des zweiten Sterilfilters 14 geht ein dritter Abschnitt 11c der Dialysierflüssigkeitsleitung 11 ab und führt zu dem Einlass 4a der Dialysierflüssigkeitskammer 4 des Dialysators 1.

Die sterile Dialysierflüssigkeit wird der zweiten Kammer 14B des zweiten Sterilfilters 14 entnommen. Von den beiden Auslässen c, d der zweiten Kammer 14B des zweiten Sterilfilters 14 geht eine Substituatleitung 15 ab, die zu dem extrakorporalen Blutkreislauf 5A führt. Die Substituatleitung 15 kann entweder an die arterielle Tropfkammer 8 oder die venöse Tropfkammer 9 angeschlossen werden, um die Substitutionsflüssigkeit stromauf oder stromab des Dialysator 1 zuzuführen. Das Substituat wird mit einer Substituatpumpe 31 gefördert.

Das Flüssigkeitssystem 5B sieht vor, dass beide Sterilfilter 13, 14 derart betrieben werden können, dass die Dialysierflüssigkeit durch die semipermeable Membran der Filter strömt oder die Dialysierflüssigkeit entlang der Membran strömt. Die Strömungsrichtung durch die Membran wird als transversaler Fluss und die Strömungsrichtung entlang der Membran als longitudinaler Fluss bezeichnet.

Um den ersten Sterilfilter im Durchfluss betreiben zu können, geht von dem zweiten Einlass b der ersten Kammer 13A des Sterilfilters 13 eine Spülleitung 16 ab, die zu der Dialysierflüssigkeitsrückführleitung 12 führt. In der Spülleitung 16 befindet sich ein elektro-magnetisch oder pneumatisch betätigbares Absperrorgan 17. Weitere Absperrorgane 18, 19 befinden sich in der Dialysierflüssigkeitszuführleitung 11 zwischen dem zweiten Sterilfilter 14 und der Dialysierflüssigkeitskammer 4 des Dialysators 1 und stromab des Dialysators in der Dialysierflüssigkeitsrückführleitung 12.

Die Steuerung der Blutbehandlungsvorrichtung erfolgt mit einer zentralen Steuereinheit 20, die über Steuerleitungen mit den einzelnen Komponenten der Blutbehandlungsvorrichtung verbunden ist. In Fig. 3 sind nur die Steuerleitungen 31', 17'. 18' und 19' für die Substituatpumpe 31 sowie die Absperrorgane 17, 18 und 19 dargestellt.

Die extrakorporale Blutbehandlungsvorrichtung verfügt über eine Vorrichtung zum Überprüfen beider Sterilfilter 13, 14. Die Vorrichtung zum Überprüfen der Sterilfilter kann Bestandteil der Blutbehandlungsvorrichtung oder eine separate Vorrichtung sein. Bei dem vorliegenden Ausführungsbeispiel ist die Vorrichtung Bestandteil der Blutbehandlungsvorrichtung. Daher kann die Vorrichtung von Komponenten Gebrauch machen, die bereits in der Blutbehandlungsvorrichtung vorhanden sind.

Die Vorrichtung zur Überwachung der Sterilfilter weist eine Rechen- und Auswerteinheit 21 auf, die über eine Datenleitung 22 mit der zentralen Steuereinheit 20 verbunden ist. Die Rechen- und Auswerteinheit 21 kann aber auch Bestandteil der Steuereinheit 20 sein. Darüber hinaus weist die Vorrichtung mehrere Leitfähigkeitssensoren 25, 26, 27 und 28 auf, von denen der erste Leitfähigkeitssensor 25 in der Spülleitung 16 stromauf des Absperrorgans 17, der zweite Leitfähigkeitssensor 26 in dem zweiten Abschnitt 11b der Dialysierflüssigkeitszuführleitung 11 zwischen dem ersten und zweiten Sterilfilter 13, 14, der dritte Leitfähigkeitssensor 27 in dem dritten Abschnitt 11c der Dialysierflüssigkeitszuführleitung 11 zwischen dem zweiten Sterilfilter 14 und der Dialysierflüssigkeitskammer 4 des Dialysators 1 und der dritte Leitfähigkeitssensor 28 in der Substituatleitung 15 stromauf der Substituatpumpe 31 angeordnet ist. Die Leitfähigkeitssensoren 25, 26, 27 und 28 sind über Datenleitungen 25', 26', 27' und 28' mit der Rechen- und Auswerteinheit 21 verbunden. Des Weiteren weist die Vorrichtung eine Signaleinheit 29 auf, die über eine Datenleitung 30 mit der Rechen- und Auswerteinheit 21 verbunden ist.

Nachfolgend wird die Funktionsweise der Vorrichtung zur Überwachung der Sterilfilter im Einzelnen beschrieben.

Zum Überprüfen der Sterilfilter wird die Strömungsrichtung in den zu überprüfenden Sterilfiltern umgeschaltet. Es ist möglich, die Strömungsrichtung von einem transversalen Fluss auf einen longitudinalen Fluss oder von einem longitudinalen Fluss auf einen transversalen Fluss umzuschalten. Vorzugsweise wird die Strömungsrichtung beim Testen periodisch umgeschaltet.

Zum Überprüfen des ersten Sterilfilters 13 erfolgt die Umschaltung der Strömungsrichtung wie folgt. Für einen transversalen Fluss schließt die Steuereinheit 20 das Absperrorgan 17 in der Spülleitung 16. Die Leitfähigkeit der Dialysierflüssigkeit stromab des ersten Sterilfilters 13 wird mit dem Leitfähigkeitssensor 26 in dem zweiten Abschnitt 11b der Dialysierflüssigkeitszuführleitung 11 gemessen. Zur Erzeugung eines longitudinalen Flusses öffnet die Steuereinheit 20 das Absperrorgan 17 in der Spülleitung 16, wobei die Leitfähigkeit der Dialysierflüssigkeit mit dem Leitfähigkeitssensor 25 in der Spülleitung 16 gemessen wird.

Zur Überprüfung des zweiten Sterilfilters 14 erfolgt die Umschaltung der Strömungsrichtung wie folgt. Die Steuereinheit 20 hält zur Erzeugung eines longitudinalen Flusses die Substituatpumpe 31 an, wobei das Absperrorgan 18 in dem dritten Leitungsabschnitt 11c der Dialysierflüssigkeitszuführleitung 11 geöffnet ist. Die Dialysierflüssigkeit strömt somit durch die erste Kammer 14A des zweiten Sterilfilters 14, wobei die Leitfähigkeit der Dialysierflüssigkeit (Substituat) stromab des Sterilfilters 14 mit dem Leitfähigkeitssensor 27 gemessen wird, der in dem dritten Abschnitt 11c der Dialysierflüssigkeitszuführleitung 11 angeordnet ist. Zur Erzeugung eines transversalen Flusses schließt die Steuereinheit 20 das Absperrorgan 18 und setzt die Substituatpumpe 31 in Gang. Die Leitfähigkeit der Dialysierflüssigkeit wird mit dem Leitfähigkeitssensor 28 in der Substituatleitung 15 gemessen.

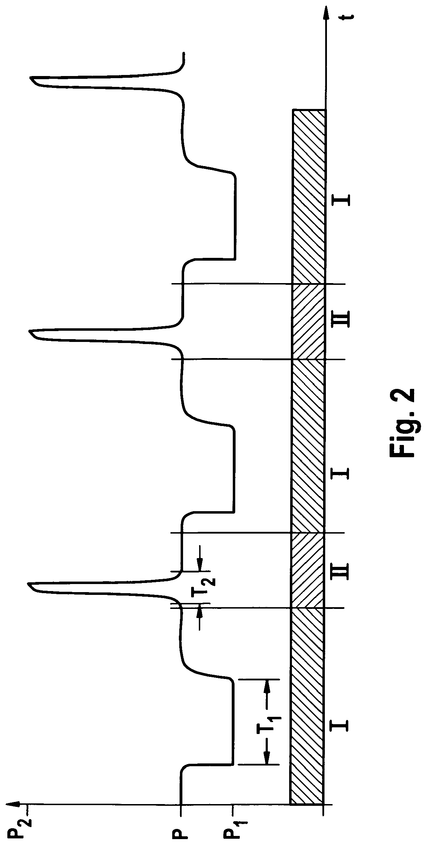

Fig. 2 zeigt die mit den beiden Leitfähigkeitssensoren 25, 26 bzw. 27, 28 gemessenen Leitfähigkeitswerte als Funktion der Zeit t, wobei periodisch die Strömungsrichtung von einem transversalen Fluss I auf einen longitudinalen Fluss II umgeschaltet wird. Fig. 2 dient nur der Veranschaulichung der Leitfähigkeitsänderung bei der periodischen Umkehr der Strömungsrichtung durch eine der beiden Filter 13, 14. In Abhängigkeit von dem Zustand der beiden Filter ergeben sich in der Praxis unterschiedliche Leitfähigkeitswerte.

Es zeigt sich, dass nach einer Umkehr der Strömungsrichtung von einem longitudinalen Fluss II auf einen transversalen Fluss I die Leitfähigkeit von einem Basiswert P innerhalb eines bestimmten Zeitintervalls T₁ auf einen Minimalwert P₁ abfällt, um dann wieder auf den Basiswert P anzusteigen. Bei einem Wechsel von einem transversalen Fluss I auf einen longitudinalen Fluss II steigt die Leitfähigkeit von dem Basiswert P innerhalb eines vorgegebenen Zeitintervalls T₂ auf einen maximalen Wert P₂, um dann wieder auf den Basiswert P abzufallen. Es lassen sich also ein Leitfähigkeitsanstieg und ein Leitfähigkeitsabfall nachweisen, der darauf zurückzuführen ist, dass kurzzeitig Ladungsträger in der elektrolytischen Flüssigkeit (Dialysierflüssigkeit) frei werden. Bei einer für Permeat typischen Basisleitfähigkeit von etwa 7 µS/cm beträgt die Leitfähigkeitsverschiebung etwa µS/cm 1 - 5 .

Die erfindungsgemäße Vorrichtung erlaubt die Überprüfung sämtliche Filter, die über polare Oberflächen verfügen. Dies ist beispielsweise bei Polysulfonfiltern der Fall. Wenn sich die elektrolytische Flüssigkeit, bei der es sich bei dem vorliegenden Ausführungsbeispiel um Dialysierflüssigkeit handelt, durch die Poren der Membran hindurchtritt (transversaler Fluss), reichern sich die negativen Ionen (Co-Ionen) an der polaren Oberfläche der Hohlfasern des Polysulfonfilters an und ziehen positive Ionen der Dialysierflüssigkeit in Richtung der Oberfläche der Kapillaren. Dieser Vorgang findet nicht nur an den Poren der Kapillaren, sondern an der gesamten Oberfläche des Polysulfonfilters statt. Die Innenwände der Poren werden also von positiven Ionen der Dialysierflüssigkeit besetzt, die ihrerseits ein Gegenfeld erzeugen, das im weiteren zeitlichen Verlauf die anziehende Wirkung der Co-Ionen aufhebt, so dass sich die Felder neutralisieren. Damit kommt es zu einer Sättigung, nach der keine weiteren positiven Ionen der Dialysierflüssigkeit mehr an der Oberfläche der Kapillaren anhaften. Da die Ladungsträger aufgehalten werden, nimmt die Leitfähigkeit der Flüssigkeit stromab des Filters ab. Als Folge der Sättigung steigt dann die Leitfähigkeit wieder auf den ursprünglichen Wert (Basiswert) an. Schließlich wird die Leitfähigkeit von frischer Dialysierflüssigkeit ohne Ladungsträgerentzug gemessen.

Wenn die elektrolytische Flüssigkeit entlang der Membran des Filters strömt, werden die Poren der Kapillaren gespült. Hierdurch bewegen sich die positiven Ionen der elektrolytischen Flüssigkeit, beispielsweise der Dialysierflüssigkeit, nunmehr senkrecht zur bisherigen Richtung durch die Kapillaren. Die Co-Ionen in den Poren werden hierdurch in Richtung des Flüssigkeitsstroms zur Innenwand der Kapillaren bewegt und führen die anhaftenden positiven Ionen aus den Poren in die Kapillaren zurück. Der Dialysierflüssigkeit wird somit ein "Ladungsträgerbolus" verabreicht, der sich stromab des Filters in longitudinaler Richtung als kurzfristige Leitfähigkeitserhöhung nachweisen lässt. Nachdem dieser Ladungsträgerbolus den Leitfähigkeitssensor passiert hat, strömt wieder Dialysierflüssigkeit mit der Basisleitfähigkeit an dem Leitfähigkeitssensor vorbei.

Der Betrag des Anstiegs bzw. Abfalls der Leitfähigkeit hängt entscheidend von der Größe der Poren der Kapillaren und von der Leitfähigkeit der elektrolytischen Flüssigkeit ab. Die Größe der Poren bestimmt, wie viele Ladungsträger im Verhältnis zur durchströmenden Flüssigkeit zurückgehalten werden. Ist die Pore sehr groß, fällt die Anzahl der zurückgehaltenen Ladungsträger im Verhältnis zu der durch den großen Porendurchmesser starken Durchströmung nicht ins Gewicht. In der Praxis haben aber die Poren der Kapillaren der verwendeten Sterilfilter eine Größe, so dass sich der Leitfähigkeitsanstieg bzw. -abfall mit ausreichender Genauigkeit messen lässt.

Es hat sich gezeigt, dass sich der Leitfähigkeitssprung nach dem Wechsel der Strömungsrichtung im Filter mit zunehmendem Alter des Filters verringert. Damit kann also überprüft werden, ob der Filter auszutauschen ist.

Die Rechen- und Auswerteinheit 21 der Vorrichtung zur Überprüfung einer der beiden oder beider Filter 13, 14 der Blutbehandlungsvorrichtung misst bei einem ersten Ausführungsbeispiel den Betrag der Leitfähigkeit, um den die Leitfähigkeit von dem Basiswert P auf den Minimalwert P₁ innerhalb des vorgegebenen Zeitintervalls T₁ abfällt. Diesen Wert vergleicht die Rechen- und Auswerteinheit 21 mit einem vorgegebenen Grenzwert. Wenn der Betrag des Leitfähigkeitsabfalls kleiner als der Grenzwert ist, schließt die Rechen- und Auswerteinheit 21 darauf, dass der Filter auszutauschen ist. Die Rechen- und Auswerteinheit erzeugt dann ein Signal, das die Signaleinheit 29 empfängt. Dann wird die Aufforderung zum Austauschen des Filters mit der Signaleinheit 29 signalisiert, die einen akustischen und/oder optischen und/oder taktilen Alarm geben kann.

Der vorgegebene Grenzwert zur Beurteilung des Alters des Filters kann mit einer Referenzmessung mit einem neuen Filter überprüft werden. Bei einem Ausführungsbeispiel der Erfindung nimmt die Überwachungsvorrichtung die Referenzmessung zur Bestimmung des Grenzwertes nach dem Austausch eines alten Filters gegen einen neuen selbst vor, wobei der Grenzwert dann ein bestimmter prozentualer Anteil des Betrages der Erhöhung der Leitfähigkeit ist, der mit dem neuen Filter gemessen wird. Dieser Referenzwert wird in einem Speicher der Rechen- und Auswerteinheit 21 gespeichert. In den darauffolgenden Behandlungen werden die gemessenen Leitfähigkeitswerte dann mit dem abgespeicherten Grenzwert verglichen. Es ist auch möglich, den Grenzwert auf der Grundlage einer Messung mit einem gebrauchten Filters empirisch zu ermitteln, wobei angenommen wird, dass der gebrauchte Filter seine maximale Lebensdauer gerade erreicht hat.

Eine andere Ausführungsform der Erfindung sieht vor, nicht den negativen Leitfähigkeitsprung nach einem Wechsel der Strömungsrichtung von einem longitudinalen auf einen transversalen Fluss, sondern die Messung des Betrages des Leitfähigkeitssprungs von einem Basiswert P auf den Maximalwert P₂ nach einem Wechsel von einem transversalen auf einen longitudinalen Fluss vor. Bei diesem Ausführungsbeispiel wird der Betrag des Leitfähigkeitsanstiegs mit einem vorgegebenen Grenzwert verglichen. Es ist aber auch möglich beide Messungen miteinander zu kombinieren, der entsprechend dem Grenzwert vor den Leitfähigkeitsabfall ermittelt werden kann.

Bei einer alternativen Ausführungsform wird nicht der Betrag des Leitfähigkeitsanstiegs bzw. -abfalls, sondern das Integral des Leitfähigkeitssprungs von der Rechen- und Auswerteinheit 21 berechnet. Das Integral des Leitfähigkeitssprungs entspricht in Bezug auf den Basiswert der Fläche unter der Kurve in dem Zeitintervall T₁ bzw. T₂. Bei diesem Ausführungsbeispiel vergleicht die Rechen- und Auswerteinheit 21 das Integral des Leitfähigkeitssprungs mit einem vorgegebenen Grenzwert, der wie bei dem ersten Ausführungsbeispiel ermittelt und in dem Speicher der Rechen- und Auswerteinheit abgelegt wird.

Für den Fall, dass die Rechen- und Auswerteinheit 21 den Leitfähigkeitssprung nicht erkennt, wird auf einen möglichen Defekt des Filters geschlossen. Es hat sich gezeigt, dass bei Rissen in den Kapillaren des Filters der Durchstrom so groß ist, dass die zurückgehaltenen Ionen keinen Effekt auf die Leitfähigkeit haben. Der mögliche Defekt des Filters wird wieder mit der Signaleinheit 29 signalisiert.

Mit der Messung der Leitfähigkeit kann die Rechen- und Auswerteinheit 21 auch überprüfen, ob ein Filter zum Filtern von Dialysierflüssigkeit oder nur ein Ersatzfilter (Dummyfilter) in die Blutbehandlungsvorrichtung eingesetzt ist, der lediglich zur Herstellung einer Flüssigkeitsverbindung zwischen den Anschlüssen der Dialysierflüssigkeitsleitungen dient. Für den Fall, dass die Rechen- und Auswerteinheit 21 keinen Leitfähigkeitssprung erkennt, signalisiert die Signaleinheit 29, dass möglicherweise ein Ersatzfilter angelegt ist.

Bei einem weiteren Ausführungsbeispiel der Erfindung werden jeweils Messungen für den ersten und den zweiten Sterilfilter 13, 14 durchgeführt. Der Betrag des Leitfähigkeitssprungs bzw. das Integral des Leitfähigkeitssprungs des einen Filters vergleicht die Auswerteinheit dann mit dem Betrag bzw. dem Integral des Leitfähigkeitssprungs des anderen Filters. Wenn die Differenz der Leitfähigkeitssprünge beider Filter einen vorgegebenen Grenzwert übersteigt, wird auf unterschiedliche Abnutzungsgrade oder mögliche Defekte eines der beiden Filter geschlossen. Auch dieses Messergebnis kann wieder mit der Signaleinheit signalisiert werden.

Bei der Überprüfung von Filtern einer Hämo(dia)filtrationsvorrichtung bietet sich an, als elektrolytische Flüssigkeit das Permeat zu verwenden, das ohnehin durch das Flüssigkeitssystem strömt. Die Messung wird vorteilhaft vor der Dialysebehandlung, durchgeführt. Es ist grundsätzlich aber auch eine Messung während der Dialyse möglich, wenn der extrakorporale Blutkreislauf für den Zeitraum der Messung vom vaskulären Blutkreislauf mit einer arteriellen und venösen Klemme abgetrennt wird. Vor der eigentlichen Dialysebehandlung kann die Messung während des Spülvorgangs in der Maschine erfolgen. Die Messung vor der Dialysebehandlung hat den Vorteil, dass die Basisleitfähigkeit der Dialysierflüssigkeit (Spülflüssigkeit) ohne Rücksicht auf den Patienten verändert werden kann. Es hat sich gezeigt, dass für verschiedene Filter typische Basisleitfähigkeiten vorteilhaft sind, da sie zu einem besonders großen Leitfähigkeitssprung führen. Während der Dialyse kann die Basisleitfähigkeit bei Abkopplung des vaskulären Blutkreislaufs vom extrakorporalen Blutkreislauf zwar auch in gewissen Grenzen variiert werden. Dann muss aber die für den Patienten möglicherweise gesundheitsgefährdende Lösung erst wieder aus dem Flüssigkeitssystem entfernt werden, wobei das Flüssigkeitssystem wieder gespült und die Flüssigkeit verworfen werden muss. Es hat sich gezeigt, dass bei der Desinfizierung und Entfettung der Dialysemaschine mit Hypochlorid die elektrochemischen Eigenschaften der Oberfläche des Pyrogenfilters derart verändert werden, dass sie den Effekt der Leitfähigkeitsverschiebung verstärken.

Fig. 4 zeigt den Einfluss der Spülung mit Hypochlorid bei der Maschinendesinfektion auf die elektrisch-polaren Eigenschaften der PVP-Polysulfan-Membran. Hypochlorid wäscht das PVP (Polyvinylpyrrolidon) aus der Membran. Hierdurch wird die Membran polarer, wodurch der Effekt der Leitfähigkeitsverschiebung verstärkt wird. In der Praxis findet eine Spülung der Maschine mit Hypochlorid allerdings nur sehr selten, in der Regel 1-2 mal pro Jahr statt. Die Leitfähigkeitsverschiebung infolge der Maschinendesinfektion mit Hypchlorid kann bei der Vorgabe des Grenzwerts entsprechend berücksichtigt werden.

Die Desinfektion der Blutbehandlungsvorrichtung mit einem temperierten Desinfektionsmittel, beispielsweise einer Citratlösung, hat hingegen keinen Einfluss auf die Größe der Leitfähigkeitsverschiebung.

## Patentansprüche

1. Vorrichtung zur Überprüfung eines Filters, der eine semipermeable Membran aufweist, die Primärseite und Sekundärseite des Filters voneinander trennt, **dadurch gekennzeichnet, dass** die Vorrichtung aufweist:
Mittel (20; 25 - 28) zum Wechsel der Strömungsrichtung zwischen einem transversalen Fluss einer elektrolytischen Flüssigkeit durch die semipermeable Membran des Filters und einem longitudinalen Fluss der elektrolytischen Flüssigkeit entlang der semipermeablen Membran des Filters oder ein Wechsel zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters,
Mittel (20; 16, 17, 18, 19) zum Messen der Leitfähigkeit der in transversaler Richtung strömenden elektrolytischen Flüssigkeit stromab des Filters und/oder der in longitudinaler Richtung strömenden elektrolytischen Flüssigkeit stromab des Filters, und
eine Rechen- und Auswerteinheit (21) zur Überwachung der Änderung der Leitfähigkeit nach dem Wechsel der Strömungsrichtung der elektrolytischen Flüssigkeit von einem Basiswert, die derart konfiguriert ist, dass ein nicht ordnungsgemäßer Zustand des Filters auf der Grundlage der Änderung der Leitfähigkeit nach dem Wechsel der Strömungsrichtung der elektrolytischen Flüssigkeit festgestellt wird;
charakterisiert durch eine Steuereinheit (20), die mit den Mitteln zum Wechsel der Strömungsrichtung, mit den Mitteln zum Messen der Leitfähigkeit sowie der Rechen- und Auswerteinheit (21) verbunden ist und die konfiguriert ist zur Durchführung eines Verfahrens zur Überprüfung des Filters mit folgenden Verfahrensschritten:
Erzeugen eines Wechsels der Strömungsrichtung zwischen einem transversalen Fluss einer elektrolytischen Flüssigkeit durch die semipermeable Membran des Filters und einem longitudinalen Fluss der elektrolytischen Flüssigkeit entlang der semipermeablen Membran des Filters oder eines Wechsels zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters,
Messen der Leitfähigkeit der in transversaler Richtung strömenden elektrolytischen Flüssigkeit stromab des Filters und/oder der in longitudinaler Richtung strömenden elektrolytischen Flüssigkeit stromab des Filters,
Überwachen der Änderung der Leitfähigkeit nach dem Wechsel der Strömungsrichtung der elektrolytischen Flüssigkeit von einem Basiswert und
Schließen auf einen nicht ordnungsgemäßen Zustand des Filters auf der Grundlage der Änderung der Leitfähigkeit nach dem Wechsel der Strömungsrichtung der elektrolytischen Flüssigkeit.

2. Vorrichtung nach Anspruch 1, **dadurch gekennzeichnet, dass** die Rechen- und Auswerteinheit (21), derart konfiguriert ist, dass nach einem Wechsel der Strömungsrichtung einer elektrolytischen Flüssigkeit zwischen einem transversalen Fluss durch die semipermeable Membran des Filters und einem longitudinalen Fluss der elektrolytischen Flüssigkeit entlang der semipermeablen Membran des Filters der Betrag der Veränderung der Leitfähigkeit von einem Basiswert ermittelt wird und mit einem vorgegebenen Grenzwert verglichen wird, wobei ein nicht ordnungsgemäßer Zustand des Filters festgestellt wird, wenn der Betrag kleiner als der vorgegebene Grenzwert ist.

3. Vorrichtung nach Anspruch 1 oder 2, **dadurch gekennzeichnet, dass** die Rechen- und Auswerteinheit (21), derart konfiguriert ist, dass nach einem Wechsel der Strömungsrichtung einer elektrolytischen Flüssigkeit zwischen einem longitudinalen Fluss entlang der semipermeablen Membran des Filters und einem transversalen Fluss der elektrolytischen Flüssigkeit durch die semipermeable Membran des Filters der Betrag der Veränderung der Leitfähigkeit von einem Basiswert ermittelt wird und mit einem vorgegebenen Grenzwert verglichen wird, wobei ein nicht ordnungsgemäßer Zustand des Filters festgestellt wird, wenn der Betrag kleiner als der vorgegebene Grenzwert ist.

4. Vorrichtung nach Anspruch 1, **dadurch gekennzeichnet, dass** die Rechen- und Auswerteinheit (21), derart konfiguriert ist, dass nach einem Wechsel der Strömungsrichtung einer elektrolytischen Flüssigkeit zwischen einem transversalen Fluss durch die semipermeable Membran des Filters und einem longitudinalen Fluss der elektrolytischen Flüssigkeit entlang der semipermeablen Membran des Filters das Integral der Leitfähigkeitsveränderung ermittelt wird und mit einem vorgegebenen Grenzwert verglichen wird, wobei ein nicht ordnungsgemäßer Zustand des Filters festgestellt wird, wenn der Betrag kleiner als der vorgegebene Grenzwert ist.

5. Vorrichtung nach Anspruch 1 oder 2, **dadurch gekennzeichnet, dass** die Rechen- und Auswerteinheit (21), derart konfiguriert ist, dass nach einem Wechsel zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters das Integral der Leitfähigkeitsveränderung ermittelt wird und mit einem vorgegebenen Grenzwert verglichen wird, wobei ein nicht ordnungsgemäßer Zustand des Filters festgestellt wird, wenn der Betrag kleiner als der vorgegebene Grenzwert ist.

6. Vorrichtung nach einem der Ansprüche 1 bis 5, **dadurch gekennzeichnet, dass** die Rechen- und Auswerteinheit (21), derart konfiguriert ist, dass ein nicht ordnungsgemäßer Zustand des Filters festgestellt wird, wenn eine Änderung der Leitfähigkeit von dem Basiswert nicht gemessen wird.

7. Vorrichtung nach einem der Ansprüche 1 bis 6, **dadurch gekennzeichnet, dass** die elektrolytische Flüssigkeit Dialysierflüssigkeit oder Permeat ist.

8. Vorrichtung nach einem der Ansprüche 1 bis 7, **dadurch gekennzeichnet, dass** der Filter ein Kapillarfilter ist, der ein Bündel (A) von Hohlfasern aufweist.

9. Vorrichtung nach einem der Ansprüche 1 bis 8, **dadurch gekennzeichnet, dass** der Filter ein Filter (13, 14) zum Filtern von Dialysierflüssigkeit für eine extrakorporale Blutbehandlungsvorrichtung ist.

10. Vorrichtung zur extrakorporalen Blutbehandlung mit einem extrakorporalen Blutkreislauf (5A), der eine erste Kammer (3) eines durch eine semipermeable Membran (2) in die erste Kammer (3) und zweite Kammer (4) unterteilten Dialysator (1) einschließt, und einem Dialysierflüssigkeitssystem (5B), das die zweite Kammer (4) des Dialysators einschließt, wobei von dem Dialysierflüssigkeitssystem (5B) eine Substituatleitung (15) zu dem Blutkreislauf (5A) führt und in dem Flüssigkeitssystem (5A) mindestens ein Filter (13, 14) zum Filtern von Dialysierflüssigkeit angeordnet ist,
und einer Vorrichtung zur Überprüfung des mindestens einen im Flüssigkeitssystem (5A) angeordneten Filters (13, 14) zum Filtern von Dialysierflüssigkeit nach einem der Ansprüche 1 bis 9.

11. Vorrichtung nach Anspruch 10, **dadurch gekennzeichnet, dass** die Vorrichtung eine mit der Rechen- und Auswerteinheit (21) zusammenwirkende Signaleinheit (22) aufweist, die ein Signal gibt, wenn von der Rechen- und Auswerteinheit ein nicht ordnungsgemäßer Zustand des Filters festgestellt wird.

12. Verfahren zur Überprüfung eines Filters, der eine semipermeable Membran aufweist, die Primärseite und Sekundärseite des Filters voneinander trennt, mit folgenden Verfahrensschritten:
Erzeugen eines Wechsels der Strömungsrichtung zwischen einem transversalen Fluss einer elektrolytischen Flüssigkeit durch die semipermeable Membran des Filters und einem longitudinalen Fluss der elektrolytischen Flüssigkeit entlang der semipermeablen Membran des Filters oder eines Wechsels zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters,
Messen der Leitfähigkeit der in transversaler Richtung strömenden elektrolytischen Flüssigkeit stromab des Filters und/oder der in longitudinaler Richtung strömenden elektrolytischen Flüssigkeit stromab des Filters,
Überwachung der Änderung der Leitfähigkeit nach dem Wechsel der Strömungsrichtung der elektrolytischen Flüssigkeit von einem Basiswert und Schließen auf einen nicht ordnungsgemäßen Zustand des Filters auf der Grundlage der Änderung der Leitfähigkeit nach dem Wechsel der Strömungsrichtung der elektrolytischen Flüssigkeit.

13. Verfahren nach Anspruch 12, **dadurch gekennzeichnet, dass** nach einem Wechsel der Strömungsrichtung einer elektrolytischen Flüssigkeit zwischen einem transversalen Fluss durch die semipermeable Membran des Filters und einem longitudinalen Fluss der elektrolytischen Flüssigkeit entlang der semipermeablen Membran des Filters der Betrag der Veränderung der Leitfähigkeit von einem Basiswert ermittelt wird und mit einem vorgegebenen Grenzwert verglichen wird, wobei auf einen nicht ordnungsgemäßen Zustand des Filters geschlossen wird, wenn der Betrag kleiner als der vorgegebene Grenzwert ist.

14. Verfahren nach Anspruch 12 oder 13, **dadurch gekennzeichnet, dass** nach einem Wechsel der Strömungsrichtung zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters der Betrag der Veränderung der Leitfähigkeit von einem Basiswert ermittelt wird und mit einem vorgegebenen Grenzwert verglichen wird, wobei auf einen nicht ordnungsgemäßen Zustand des Filters geschlossen wird, wenn der Betrag kleiner als der vorgegebene Grenzwert ist.

15. Verfahren nach Anspruch 12 oder 13, **dadurch gekennzeichnet, dass** nach einem Wechsel der Strömungsrichtung zwischen einem transversalen Fluss einer elektrolytischen Flüssigkeit durch die semipermeable Membran des Filters und einem longitudinalen Fluss der elektrolytischen Flüssigkeit entlang der semipermeablen Membran des Filters das Integral der Leitfähigkeitsveränderung ermittelt wird und mit einem vorgegebenen Grenzwert verglichen wird, wobei auf einen nicht ordnungsgemäßen Zustand des Filters geschlossen wird, wenn der Betrag kleiner als der vorgegebene Grenzwert ist.

16. Verfahren nach Anspruch 12 oder 13, **dadurch gekennzeichnet, dass** nach einem Wechsel der Strömungsrichtung zwischen einem longitudinalen Fluss einer elektrolytischen Flüssigkeit entlang der semipermeablen Membran des Filters und einem transversalen Fluss der elektrolytischen Flüssigkeit durch die semipermeable Membran des Filters das Integral der Leitfähigkeitsveränderung ermittelt wird und mit einem vorgegebenen Grenzwert verglichen wird, wobei auf einen nicht ordnungsgemäßen Zustand des Filters geschlossen wird, wenn der Betrag kleiner als der vorgegebene Grenzwert ist.

17. Verfahren nach einem der Ansprüche 12 bis 16, **dadurch gekennzeichnet, dass** auf einen nicht ordnungsgemäßen Zustand des Filters geschlossen wird, wenn eine Änderung der Leitfähigkeit von dem Basiswert nicht gemessen wird.

18. Verfahren nach einem der Ansprüche 12 bis 17, **dadurch gekennzeichnet, dass** die elektrolytische Flüssigkeit Dialysierflüssigkeit oder Permeat ist.

19. Verfahren nach einem der Ansprüche 12 bis 18, **dadurch gekennzeichnet, dass** der Filter ein Kapillarfilter ist, der ein Bündel von Hohlfasern aufweist.

20. Verfahren nach einem der Ansprüche 12 bis 19, **dadurch gekennzeichnet, dass** der Filter ein Pyrogenfilter zum Filtern von Dialysierflüssigkeit für eine extrakorporale Blutbehandlungsvorrichtung ist.

## Claims

1. A device for checking a filter, which comprises a semipermeable membrane which separates the primary side and secondary side of the filter, **characterised in that** the device comprises
means (20; 25 - 28) for generating a change in the flow direction between a transverse flow of an electrolytic fluid through the semipermeable membrane of the filter and a longitudinal flow of the electrolytic fluid along the semipermeable membrane of the filter or a change between a longitudinal flow of an electrolytic fluid along the semipermeable membrane of the filter and a transverse flow of the electrolytic fluid through the semipermeable membrane of the filter,
means (20; 16, 17, 18, 19) for measuring the conductivity of the electrolytic fluid flowing in the transverse direction downstream of the filter and/or of the electrolytic fluid flowing in the longitudinal direction downstream of the filter and
a computing and evaluation unit (21) for monitoring the change in the conductivity after the change in the flow direction of the electrolytic fluid from a base value, said unit being configured such that an incorrectly functioning state of the filter is ascertained on the basis of the change in the conductivity after the change in the flow direction of the electrolytic fluid;
characterized through
a control unit (20), which is connected with the means for generating a change in the flow direction, the means for measuring the conductivity and the computing and evaluation unit (21) and is configured for performing a method for checking the filter comprising the following method steps:
generating a change in the flow direction between a transverse flow of an electrolytic fluid through the semipermeable membrane of the filter and a longitudinal flow of the electrolytic fluid along the semipermeable membrane of the filter or a change between a longitudinal flow of an electrolytic fluid along the semipermeable membrane of the filter and a transverse flow of the electrolytic fluid through the semipermeable membrane of the filter,
measuring the conductivity of the electrolytic fluid flowing in the transverse direction downstream of the filter and/or of the electrolytic fluid flowing in the longitudinal direction downstream of the filter,
monitoring the change in the conductivity after the change in the flow direction of the electrolytic fluid from a base value and
concluding that there is an incorrectly functioning state of the filter on the basis of the change in the conductivity after the change in the flow direction of the electrolytic fluid.

2. The device according to claim 1, **characterised in that** the computing and evaluation unit (21) is configured such that, after a change in the flow direction of an electrolytic fluid between a transverse flow through a semipermeable membrane of the filter and a longitudinal flow of the electrolytic fluid along the semipermeable membrane of the filter, the amount of the change in conductivity from a base value is ascertained and compared with a preselected threshold value, an incorrectly functioning state of the filter being ascertained if the amount is less than the preselected threshold value.

3. The device according to claim 1 or 2, **characterised in that** the computing and evaluation unit (21) is configured such that, after a change in the flow direction of an electrolytic fluid between a longitudinal flow along the semipermeable membrane of the filter and a transverse flow of the electrolytic fluid through the semipermeable membrane of the filter, the amount of the change in conductivity from a base value is ascertained and compared with a preselected threshold value, an incorrectly functioning state of the filter being ascertained if the amount is less than the preselected threshold value.

4. The device according to claim 1, **characterised in that** the computing and evaluation unit (21) is configured such that, after a change in the flow direction of an electrolytic fluid between a transverse flow through the semipermeable membrane of the filter and a longitudinal flow of the electrolytic fluid along the semipermeable membrane of the filter, the integral of the change in conductivity is ascertained and compared with a preselected threshold value, an incorrectly functioning state of the filter being ascertained if the amount is less than the preselected threshold value.

5. The device according to claim 1 or 2, **characterised in that** the computing and evaluation unit (21) is configured such that, after a change between a longitudinal flow of an electrolytic fluid along the semipermeable membrane of the filter and a transverse flow of the electrolytic fluid through the semipermeable membrane of the filter, the integral of the change in conductivity is ascertained and compared with a preselected threshold value, an incorrectly functioning state of the filter being ascertained if the amount is less than the preselected threshold value.

6. The device according to any one of claims 1 to 5, **characterised in that** the computing and evaluation unit (21) is configured such that an incorrectly functioning state of the filter is ascertained if a change in the conductivity from the base value is not measured.

7. The device according to any one of claims 1 to 6, **characterised in that** the electrolytic fluid is dialysing fluid or permeate.

8. The device according to any one of claims 1 to 7, **characterised in that** the filter is a capillary filter, which comprises a bundle (A) of hollow fibres.

9. The device according to any one of claims 1 to 8, **characterised in that** the filter is a filter (13, 14) for filtering dialysing fluid for an extracorporeal blood treatment apparatus.

10. A device for extracorporeal blood treatment with
an extracorporeal blood circuit (5A), which includes a first chamber (3) of a dialyser (1) divided by a semipermeable membrane (2) into the first chamber (3) and a second chamber (4), and a dialysing fluid system (5B), which includes the second chamber (4) of the dialyser, wherein a substituate line (15) leads from the dialysing fluid system (5B) to the blood circuit (5A) and at least one filter (13, 14) for filtering dialysing fluid is disposed in the fluid system (5A),
and a device according to any one of claims 1 to 9 for checking the at least one filter (13, 14) for filtering dialysing fluid, said filter being disposed in the fluid system (5A).

11. The device according to claim 10, **characterised in that** the device comprises a signal unit (22), which cooperates with the computing and evaluation unit (21) and emits a signal when an incorrectly functioning state of the filter is ascertained by the computing and evaluation unit.

12. A method for checking a filter, which comprises a semipermeable membrane which separates the primary side and secondary side of the filter from one another, with the following process steps:
generating a change in the flow direction between a transverse flow of an electrolytic fluid through the semipermeable membrane of the filter and a longitudinal flow of the electrolytic fluid along the semipermeable membrane of the filter or a change between a longitudinal flow of an electrolytic fluid along the semipermeable membrane of the filter and a transverse flow of the electrolytic fluid through the semipermeable membrane of the filter, measuring the conductivity of the electrolytic fluid flowing in the transverse direction downstream of the filter and/or of the electrolytic fluid flowing in the longitudinal direction downstream of the filter,
monitoring the change in the conductivity after the change in the flow direction of the electrolytic fluid from a base value and
concluding that there is an incorrectly functioning state of the filter on the basis of the change in the conductivity after the change in the flow direction of the electrolytic fluid.

13. The method according to claim 12, **characterised in that**, after a change in the flow direction of an electrolytic fluid between a transverse flow through the semipermeable membrane of the filter and a longitudinal flow of the electrolytic fluid along the semipermeable membrane of the filter, the amount of the change in conductivity from a base value is ascertained and compared with a preselected threshold value, it being concluded that there is an incorrectly functioning state of the filter if the amount is less than the preselected threshold value.

14. The method according to claim 12 or 13, **characterised in that**, after a change in the flow direction between a longitudinal flow of an electrolytic fluid along the semipermeable membrane of the filter and a transverse flow of the electrolytic fluid through the semipermeable membrane of the filter, the amount of the change in conductivity from a base value is ascertained and compared with a preselected threshold value, it being concluded that there is an incorrectly functioning state of the filter if the amount is less than the preselected threshold value.

15. The method according to claim 12 or 13, **characterised in that**, after a change in the flow direction between a transverse flow of an electrolytic fluid through the semipermeable membrane of the filter and a longitudinal flow of the electrolytic fluid along the semipermeable membrane of the filter, the integral of the change in conductivity is ascertained and compared with a preselected threshold value, it being concluded that there is an incorrectly functioning state of the filter if the amount is less than the preselected threshold value.

16. The method according to claim 12 or 13, **characterised in that**, after a change in the flow direction between a longitudinal flow of an electrolytic fluid along the semipermeable membrane of the filter and a transverse flow of the electrolytic fluid through the semipermeable membrane of the filter, the integral of the change in conductivity is ascertained and compared with a preselected threshold value, it being concluded that there is an incorrectly functioning state of the filter if the amount is less than the preselected threshold value.

17. The method according to any one of claims 12 to 16, **characterised in that** it is concluded that there is an incorrectly functioning state of the filter if a change in the conductivity from the base value is not measured.

18. The method according to any one of claims 12 to 17, **characterised in that** the electrolytic fluid is dialysing fluid or permeate.

19. The method according to any one of claims 12 to 18, **characterised in that** the filter is a capillary filter, which comprises a bundle of hollow fibres.

20. The method according to any one of claims 12 to 19, **characterised in that** the filter is a pyrogen filter for filtering dialysing fluid for an extracorporeal blood treatment apparatus.

## Revendications

1. Dispositif servant à contrôler un filtre, qui présente une membrane semi-perméable, qui sépare l'un de l'autre le côté primaire et le côté secondaire du filtre, **caractérisé en ce que** le dispositif présente :
des moyens (20 ; 25 - 28) servant à changer la direction d'écoulement entre un flux transversal d'un liquide électrolytique à travers la membrane semi-perméable du filtre et un flux longitudinal du liquide électrolytique le long de la membrane semi-perméable du filtre ou pour un changement entre un flux longitudinal d'un liquide électrolytique le long de la membrane semi-perméable du filtre et un flux transversal du liquide électrolytique à travers la membrane semi-perméable du filtre,
des moyens (20 ; 16, 17, 18, 19) servant à mesurer la conductivité du liquide électrolytique circulant dans la direction transversale en aval du filtre et/ou du liquide électrolytique circulant dans la direction longitudinale en aval du filtre, et
une unité de calcul et d'analyse (21) servant à contrôler la modification de la conductivité après le changement de la direction d'écoulement du liquide électrolytique depuis une valeur de base, qui est configurée de telle manière qu'un état non conforme du filtre est constaté sur la base de la modification de la conductivité après le changement de la direction d'écoulement du liquide électrolytique ;
**caractérisé par** une unité de commande (20), qui est reliée aux moyens servant à changer la direction d'écoulement, aux moyens servant à mesurer la conductivité ainsi qu'à l'unité de calcul et d'analyse (21) et qui est configurée pour mettre en oeuvre un procédé servant à contrôler le filtre, avec des étapes de procédé suivantes de :
production d'un changement de la direction d'écoulement entre un flux transversal d'un liquide électrolytique à travers la membrane semi-perméable du filtre et un flux longitudinal du liquide électrolytique le long de la membrane semi-perméable du filtre ou un changement entre un flux longitudinal d'un liquide électrolytique le long de la membrane semi-perméable du filtre et un flux transversal du liquide électrolytique à travers la membrane semi-perméable du filtre,
mesure de la conductivité du liquide électrolytique circulant dans la direction transversale en aval du filtre et/ou du liquide électrolytique circulant dans la direction longitudinale en aval du filtre,
contrôle de la modification de la conductivité après le changement de la direction d'écoulement du liquide électrolytique depuis une valeur de base et
conclusion d'un état non conforme du filtre sur la base de la modification de la conductivité après le changement de la direction d'écoulement du liquide électrolytique,

2. Dispositif selon la revendication 1, **caractérisé en ce que** l'unité de calcul et d'analyse (21) est configurée de telle manière qu'après un changement de la direction d'écoulement d'un liquide électrolytique entre un flux transversal à travers la membrane semi-perméable du filtre et un flux longitudinal du liquide électrolytique le long de la membrane semi-perméable du filtre, la valeur de la modification de la conductivité est déterminée depuis une valeur de base et est comparée à une valeur limite prédéfinie, dans lequel un état non conforme du filtre est constaté quand la valeur est inférieure à la valeur limite prédéfinie.

3. Dispositif selon la revendication 1 ou 2, **caractérisé en ce que** l'unité de calcul et d'analyse (21) est configurée de telle manière qu'après un changement de la direction d'écoulement d'un liquide électrolytique entre un flux longitudinal le long de la membrane semi-perméable du filtre et un flux transversal du liquide électrolytique à travers la membrane semi-perméable du filtre, la valeur de la modification de la conductivité est déterminée depuis une valeur de base et est comparée à une valeur limite prédéfinie, dans lequel un état non conforme du filtre est constaté quand la valeur est inférieure à la valeur limite prédéfinie.

4. Dispositif selon la revendication 1, **caractérisé en ce que** l'unité de calcul et d'analyse (21) est configurée de telle manière qu'après un changement de la direction d'écoulement d'un liquide électrolytique entre un flux transversal à travers la membrane semi-perméable du filtre et un flux longitudinal du liquide électrolytique le long de la membrane semi-perméable du filtre, l'intégrale de la modification de conductivité est déterminée et est comparée à une valeur limite prédéfinie, dans lequel un état non conforme du filtre est constaté quand la valeur est inférieure à la valeur limite prédéfinie.

5. Dispositif selon la revendication 1 ou 2, **caractérisé en ce que** l'unité de calcul et d'analyse (21) est configurée de telle manière qu'après un changement entre un flux longitudinal d'un liquide électrolytique le long de la membrane semi-perméable du filtre et un flux transversal du liquide électrolytique à travers la membrane semi-perméable du filtre, l'intégrale de la modification de conductivité est déterminée et est comparée à une valeur limite prédéfinie, dans lequel un état non conforme du filtre est constaté quand la valeur est inférieure à la valeur limite prédéfinie.

6. Dispositif selon l'une quelconque des revendications 1 à 5, **caractérisé en ce que** l'unité de calcul et d'analyse (21) est configurée de telle manière qu'un état non conforme du filtre est constaté quand une modification de la conductivité depuis la valeur de base n'est pas mesurée.

7. Dispositif selon l'une quelconque des revendications 1 à 6, **caractérisé en ce que** le liquide électrolytique est un liquide de dialyse ou un perméat.

8. Dispositif selon l'une quelconque des revendications 1 à 7, caractérisé e ce que le filtre est un filtre capillaire, qui présente un faisceau (A) de fibres creuses.

9. Dispositif selon l'une quelconque des revendications 1 à 8, **caractérisé en ce que** le filtre est un filtre (13, 14) servant à filtrer du liquide de dialyse pour un dispositif de traitement extracorporel du sang.

10. Dispositif servant au traitement extracorporel du sang avec
un circuit de sang (5A) extracorporel, qui renferme une première chambre (3) d'un dialyseur (1) divisé par une membrane semi-perméable (2) en la première chambre (3) et la deuxième chambre (4), et un système de liquide de dialyse (5B), qui renferme la deuxième chambre (4) du dialyseur, dans lequel un conduit de substitut (15) mène depuis le système de liquide de dialyse (5B) au circuit de sang (5A) et au moins un filtre (13, 14) servant à filtrer le liquide de dialyse est disposé dans le système de liquide (5A),
et un dispositif servant à contrôler l'au moins un filtre (13, 14) disposé dans le système de liquide (5A), servant à filtrer du liquide de dialyse selon l'une quelconque des revendications 1 à 9.

11. Dispositif selon la revendication 10, **caractérisé en ce que** le dispositif présente une unité de signal (22) coopérant avec l'unité de calcul et d'analyse (21), qui émet un signal quand un état non conforme du filtre est constaté par l'unité de calcul et d'analyse.

12. Procédé servant à contrôler un filtre, qui présente une membrane semi-perméable, qui sépare l'un de l'autre le côté primaire et le côté secondaire du filtre, avec des étapes de procédé suivantes de :
production d'un changement de la direction d'écoulement entre un flux transversal d'un liquide électrolytique à travers la membrane semi-perméable du filtre et un flux longitudinal du liquide électrolytique le long de la membrane semi-perméable du filtre ou d'un changement entre un flux longitudinal d'un liquide électrolytique le long de la membrane semi-perméable du filtre et un flux transversal du liquide électrolytique à travers la membrane semi-perméable du filtre,
mesure de la conductivité du liquide électrolytique circulant dans la direction transversale en aval du filtre et/ou du liquide électrolytique circulant dans la direction longitudinale en aval du filtre, et
contrôle de la modification de la conductivité après le changement de la direction d'écoulement du liquide électrolytique depuis une valeur de base, et
conclusion d'un état non conforme du filtre sur la base de la modification de la conductivité après le changement de la direction d'écoulement du liquide électrolytique.

13. Procédé selon la revendication 12,
**caractérisé en ce qu'**après un changement de la direction d'écoulement d'un liquide électrolytique entre un flux transversal à travers la membrane semi-perméable du filtre et un flux longitudinal du liquide électrolytique le long de la membrane semi-perméable du filtre, la valeur de la modification de la conductivité est déterminée depuis une valeur de base et est comparée à une valeur limite prédéfinie, dans lequel on conclut un état non conforme du filtre quand la valeur est inférieure à la valeur limite prédéfinie.

14. Procédé selon la revendication 12 ou 13, **caractérisé en ce qu'**après un changement de la direction d'écoulement entre un flux longitudinal d'un liquide électrolytique le long de la membrane semi-perméable du filtre et un flux transversal du liquide électrolytique à travers la membrane semi-perméable du filtre, la valeur de la modification de la conductivité est déterminée depuis une valeur de base et est comparée à une valeur limite prédéfinie, dans lequel on conclut à un état non conforme du filtre quand la valeur est inférieure à la valeur limite prédéfinie.

15. Procédé selon la revendication 12 ou 13, **caractérisé en ce qu'**après un changement de la direction d'écoulement entre un flux transversal d'un liquide électrolytique à travers la membrane semi-perméable du filtre et un flux longitudinal du liquide électrolytique le long de la membrane semi-perméable du filtre, l'intégrale de la modification de conductivité est déterminée et est comparée à une valeur limite prédéfinie, dans lequel on conclut à un état non conforme du filtre quand la valeur est inférieure à la valeur limite prédéfinie.

16. Procédé selon la revendication 12 ou 13, **caractérisé en ce qu'**après un changement de la direction d'écoulement entre un flux longitudinal d'un liquide électrolytique le long de la membrane semi-perméable du filtre et un flux transversal du liquide électrolytique à travers la membrane semi-perméable du filtre, l'intégrale de la modification de conductivité est déterminée et est comparée à une valeur limite prédéfinie, dans lequel on conclut à un état non conforme du filtre quand la valeur est inférieure à la valeur limite prédéfinie.

17. Procédé selon l'une quelconque des revendications 12 à 16,
**caractérisé en ce qu'**on conclut à un état non conforme du filtre quand une modification de la conductivité depuis la valeur de base n'est pas mesurée.

18. Procédé selon l'une quelconque des revendications 12 à 17, **caractérisé en ce que** le liquide électrolytique est un liquide de dialyse ou un perméat.

19. Procédé selon l'une quelconque des revendications 12 à 18, **caractérisé en ce que** le filtre est un filtre capillaire, qui présente un faisceau de fibres creuses.

20. Procédé selon l'une quelconque des revendications 12 à 19, **caractérisé en ce que** le filtre est un filtre pyrogène servant à filtrer du liquide de dialyse pour un dispositif de traitement extracorporel de sang.
